# EUROPEAN PATENT SPECIFICATION

(11) **EP 1 377 277 B1**
(45) Date of publication and mention of the grant of the patent: **06.08.2008**
(21) Application number: 02706082.1
(22) Date of filing: 28.01.2002
(51) Int. Cl.: A61K 31/22, A61P 3/06

(54) **PHARMACEUTICAL COMPOSITIONS COMPRISING PRAVASTATIN FOR REDUCING LDL CHOLESTEROL LEVELS**
PRAVASTATIN UMFASSENDE ARZNEIMITTEL ZUM SENKEN VON LDL- CHOLESTERINSPIEGELN
COMPOSITIONS PHARMACEUTIQUES RENFERMANT DE LA PRAVASTATINE AUX FINS DE REDUCTION DES TAUX DE CHOLESTEROL LDL

(30) Priority: 27.02.2001 US 794449
(43) Date of publication of application: 07.01.2004
(73) Proprietor: Bristol-Myers Squibb Company, Princeton, NJ 08543 (US)
(72) Inventor: BELDER, Rene, Hopewell, NJ 08525 (US); MCGOVERN, Mark E., The Floridian - Penthouse 3, Miami Beach, FL 33139 (US)
(74) Representative: Vossius & Partner
(86) International application number: PCT/US2002/002736
(87) International publication number: WO 2002/067911

(56) References cited:
- EP-A- 0 671 171
- WO-A-01/32162
- GB-A- 2 253 787
- US-A- 5 691 375

## Description

### Field of the Invention

The present invention relates to a pharmaceutical composition, preferably in the form of a tablet, which includes a single dose of more than 40 mg of pravastatin.

### Background of the Invention

Pravastatin sodium (hereinafter referred to as pravastatin) is a 3-hydroxy-3-methylglutaryl Coenzyme A (HMG-CoA) reductase inhibitor that has been evaluated in clinical studies since 1985, and marketed as a cholesterol lowering agent since 1991. Clinical trial experience with pravastatin is extensive. Pravastatin has gained approval for slowing progression of coronary and carotid atherosclerosis in patients with hypercholesterolemia. Pravastatin has been shown to reduce the risk of myocardial infarction, coronary and cardiovascular mortality and total mortality in asymptomatic patients. Pravastatin has been well tolerated and all studies have demonstrated excellent safety experience.

Despite this background, the dose response pattern for pravastatin has not been fully explored. A standard dose of pravastatin is 40 mg/day. Studies of pravastatin have been done with total daily doses of 10-80 mg of pravastatin, but it is not believed that single doses of more than 40 mg have been studied.

Surprisingly, it has been found that single doses of more than 40 mg, and in particular single doses of 60 mg, 80 mg and 160 mg, provide unexpected reductions in LDL-cholesterol. In particular, single daily doses of 80 mg and 160 mg of pravastatin reduce LDL-cholesterol levels to a surprisingly greater degree than would have been expected based on the reductions previously seen with lower daily dosage amounts.

### Summary of the Invention

One object of the instant invention is to provide a novel use of pravastatin for the manufacture of a medicament for reducing LDL cholesterol levels in a mammal comprising the administration of a therapeutically effective amount of greater than 40 mg per day, as a single dose of pravastatin or a pharmaceutically acceptable salt or ester thereof, to a mammal in need of such treatment.

Another object of the instant invention is to provide a novel use of pravastatin for the manufacture of a medicament for reducing LDL cholesterol levels in a mammal comprising the administration of 80 mg per day, as a single dose of pravastatin or a pharmaceutically acceptable salt or ester thereof, to a mammal in need of such treatment.

Still another object of the invention is to provide a novel use of pravastatin for the manufacture of a medicament for reducing LDL cholesterol levels in a mammal comprising the administration of 160 mg per day, as a single dose of pravastatin or a pharmaceutically acceptable salt or ester thereof, to a mammal in need of such treatment.

Yet another object of the instant invention involves the above-described uses further comprising the administration of one or more additional active agents, for example, a bile acid sequestrant, cholesterol absorption inhibitor, squalene synthase inhibitor, folic acid, and/or niacin, either in separate or combined dosage formulations. A further object is to provide pharmaceutical compositions which can be used in the above-described uses. Additional objects will be evident from the following detailed description.

### Description of the Invention

In accordance with the present invention, then, a pharmaceutical composition is provided which includes a single dose of more than 40 mg of pravastatin. The pharmaceutical composition of the invention, which is preferably in the form of a tablet, includes pravastatin and conventional excipients. These excipients can be, for example, one or more fillers, such as lactose and/or microcrystalline cellulose, one or more binders, such as microcrystalline cellulose (dry binder) or polyvinylpyrrolidone (wet binder), one or more disintegrating agents such as croscarmellose sodium, one or more lubricants such as magnesium stearate, and one or more ossifying agents such as magnesium oxide. Other excipients can be used as would be known to those in the art.

Pravastatin will be present in such pharmaceutical compositions in an amount within the range of from 1 to 60%, and preferably from

3 to 50%, by weight of the composition.

As described above, the compositions of the invention may include one or more fillers or excipients. These can be present in the compositions in an amount within the range of from 5 to 90% by weight, and preferably from 10 to 80% by weight, of the composition. Examples include lactose, sugar, corn starch, modified corn starch, mannitol, sorbitol, inorganic salts such as calcium carbonate and/or cellulose derivatives such as wood cellulose and microcrystalline cellulose. One or more binders may be present in addition to or in lieu of the fillers in an amount within the range of from 5 to 35%, and preferably from 10 to 30%, by weight of the composition. Examples of such binders which are suitable for use herein include polyvinyl-pyrrolidone (molecular weight ranging from 5000 to 80,000 and preferably 40,000), lactose, starches such as corn starch, modified corn starch, sugars, gum acacia and the like, as well as a wax binder in finely powdered form (less than 500 microns) such as carnauba wax, paraffin, spermaceti, polyethylenes or microcrystalline wax.

Where the composition is to be in the form of a tablet, it may include one or more tablet disintegrants in an amount within the range of from 0.5 to 10%, and preferably from 2 to 8%, by weight of the composition. Examples include croscarmellose sodium, crospovidone, sodium starch glycolate, corn starch or microcrystalline cellulose. Additionally, one or more tableting lubricants may be included in an amount within the range of from 0.2 to 8%, and preferably from 0.5 to 2%, by weight of the composition. Examples include magnesium stearate, stearic acid, palmitic acid, calcium stearate, talc, carnauba wax and the like. Other conventional ingredients which may optionally be present include preservatives, stabilizers, anti-adherents or silica flow conditioners or glidants, such as Syloid brand silicon dioxide as well as FD&C colors.

Tablets of the invention may also include a coating layer which may comprise from 0 to 15% by weight of the tablet composition. The coating layer which is applied over the tablet core may comprise any conventional coating formulations and may include one or more film-formers or binders, such as a hydrophilicpolymer like hydroxypropylmethyl cellulose and a hydrophobic polymer like ethyl cellulose, cellulose acetate, polyvinyl alcohol-maleic anhydride copolymers, p-pinene polymers, glyceryl esters of wood resins and the like, and one or more plasticizers, such as triethyl citrate, diethyl phthalate, propylene glycol, glycerin, butyl phthlate, castor oil and the like. Both core tablets as well as coating formulations may contain aluminum lakes to provide color.

The film formers may be applied from a solvent system containing one or more solvents including water, alcohols like methyl alcohol, ethyl alcohol or isopropyl alcohol, ketones like acetone or ethylmethyl ketone, chlorinated hydrocarbons like methylene chloride, dichloroethane or 1,1,1-trichloroethane. Where a color is employed, the color may be applied together with the film former, plasticizer and/or solvent compositions.

A preferred tablet composition of the invention will include more than 40 mg of pravastatin, and preferably 60 mg, 80 mg or 160 mg.

The pharmaceutical composition of the invention may be prepared using conventional techniques as would be known to those in the art.

As used herein, the term "pharmaceutically acceptable salts" means non-toxic salts of pravastatin which can be prepared as known to those in the art.

The term "therapeutically effective amount" is intended to mean that amount of a drug or pharmaceutical agent that will elicit the biological or medical response of the mammal that is being sought by a researcher, veterinarian, medical doctor or other clinician. The term mammal includes humans. The dosage regimen utilizing pravastatin is selected in accordance with a variety of factors including type, species, age, weight, sex and medical condition of the patient; the severity of the condition to be treated; the route of administration; the renal and hepatic function of the patient; and the particular compound or salt or ester thereof employed. A consideration of these factors is well within the purview of the ordinarily skilled clinician for the purpose of determining the therapeutically effective dosage amounts to be given to a person in need of the instant therapy.

The daily dosage amount of pravastatin used with the instant methods is more than 40 mg, given as a single dose, and preferably 60 mg, 80 mg or 160 mg administered orally. The dosage amount may be given in a single oral dosage unit or in multiple oral dosage units all at once. For example, a daily dosage amount of 80 mg may be administered with a single 80 mg tablet or with multiples of 40 mg tablets co-administered concurrently.

One or more additional active agents may be combined with pravastatin in the single dosage formulation, or may be administered to the patient in separate dosage formulations, which allows for concurrent administration (i.e., co-administration at essentially the same time) or sequential administration (i.e., co-administration at separately staggered times). The additional active agent or agents may be, cholesterol lowering compounds. Examples of additional active agents which may be employed include HMG-CoA synthase inhibitors; squalene epoxidase inhibitors; squalene synthetase inhibitors (also known as squalene synthase inhibitors); acyl-coenzyme A: cholesterol acyltransferase (ACAT) inhibitors; probucol; niacin; fibrates such as clofibrate, fenofibrate, and gemfibrizil; cholesterol absorption inhibitors; bile acid sequestrants; LDL (low density lipoprotein) receptor inducers; vitamin B₆ (also known as pyridoxine) and the pharmaceutically acceptable salts thereof such as the HCI salt; vitamin B₁₂ (also known as cyanocobalamin); platelet aggregation inhibitors such as aspirin and fibrinogen receptor antagonists; beta-blockers; and anti-oxidant vitamins such as vitamin C and E and beta carotene.

Doubling the dose of any inhibitor of 3-hydroxy-3-methylglutaryl Coenzyme A reductase is generally considered to provide an additional absolute reduction in LDL cholesterol of about 6% relative to the original baseline, at least up to the currently maximum recommended doses (see Illingworth Dr, Tobert JA, "A review of clinical trials comparing HMG-CoA reductase inhibitors, "Clin Ther. 1994; 16(3):366-85; and Pedersen TR, Tobert JA, "Benefits and risks of HMG-CoA reductase inhibitors in the prevention of coronary heart disease: a reappraisal, "Drug Safety. 1996; 1:11-24). As can be seen from the example below, surprisingly, greater than 6% absolute reduction in LDL cholesterol was shown as the dose of pravastatin was doubled from 40 mg to 80 mg, and again from 80 mg to 160 mg.

Additionally, at a dose of 40 mg of pravastatin, the dose response curve appears to be leveling off. When the results of reduction in LDL cholesterol levels from the example below are plotted, surprisingly the dose response curve did not level off. See the graph following the Example.

### Example 1

A Multicenter, Double-Blind, Placebo-Controlled, Parallel Study to Determine the LDL-C Lowering Efficacy of Pravastatin Administered Once Daily Over the Dosage Range of 40 mg Through 160 mg

**Objectives**: The primary objective of this study was to evaluate the dose-related cholesterol (LDL-C) lowering efficacy of pravastatin 40 mg, 80 mg and 160 mg after 6 weeks of once-daily oral administration in subjects with primary hypercholesterolemia. Secondary objectives were : 1) to evaluate the dose-related changes in serum total cholesterol (TC), high-density lipoprotein cholesterol (HDL-C), and triglycerides (TG) concentrations after 6 weeks of once-daily oral administration of pravastatin 40 mg, 80 mg and 160 mg in subjects with primary hypercholesterolemia; 2) to estimate the dose-related changes in serum LDL-C, TC, HDL-C, and TG concentrations after 2 weeks and 4 weeks of once-daily oral administration of pravastatin 40 mg, 80 mg and 160 mg in subjects with primary hypercholesterolemia; and 3) to ascertain the safety and tolerability of the treatment regimens after 6 weeks of once-daily oral administration of pravastatin 40 mg, 80 mg and 160 mg in subjects with primary hypercholesterolemia.

### Duration of Treatment: 6 weeks

Study Design and Methodology: This study was a multicenter, randomized, double-blind placebo-controlled, parallel design trial to determine the LDL-C lowering efficacy of pravastatin over a dose range of 40-160 mg in subjects with primary hypercholesterolemia. After at least 2 weeks of dietary stabilization and discontinuation of any previous lipid-lowering agents for a minimum of 4 weeks, subjects entered into a 4-week single-blind placebo lead-in period. At the end of the placebo lead-in period, subjects with mean serum LDL-C ≥ 160 mg/dL and mean serum TG ≤ 500 mg/dL were randomized (1:1:1:1) to one of four treatment groups: pravastatin 40 mg, pravastatin 80 mg, pravastatin 160 mg or placebo. Blinded treatment was administered once-daily at bedtime for 6 weeks.

**Number of Subjects:** 190 randomized; 189 received randomized study drug, 184 completed the study.

**Diagnosis and Main Criteria for Inclusion:** Consenting men, or postmenopausal and surgically sterile women, aged 18 through 65 years were eligible to enroll in the study if they met the following criteria: 1) mean serum LDL-C ≥ 160 mg/dL (the LDL-C concentration of the lower qualifying specimen were to be within 15% of the higher qualifying specimen) and 2) mean serum TG ≤ 500 mg/dL obtained from two consecutive qualifying specimens.

**Test Product, dose, mode of administration:** pravastatin, 40 mg tablets, oral

### Criteria for Evaluation:

| | |
|---|---|
| **Primary Efficacy Outcome Measure:** | Change from baseline in calculated serum LDL-C at Week 6 of double-blind treatment. |
| **Secondary Efficacy Outcome Measures:** | • Change from baseline in serum LDL-C at Weeks 2 and 4 of double blind treatment. |
| | • Change from baseline in serum TC, HDL-C, and TG at Weeks 2,4, and 6 of double-blind treatment. |
| **Safety**: | Medical history, physical examinations, laboratory analyses, and clinical adverse events (AEs). |

### Statistical Methods:

| | |
|---|---|
| **Changes from Baseline:** | Analyses of covariance with terms for treatment and baseline. |
| **Dose Response:** | A contrast of the dose groups from the analysis of covariance model. |

### Study Population:

Demographic characteristics for all randomized subjects, and baseline lipid measurements are presented in the following table:

**Demographic Characteristics and Baseline Efficacy Measures of Randomized Subjects with at Least One Post Randomization Visit**

| | Placebo (N=44) | Prava 40 mg (N=45) | Prava 80 mg (N=49) | Prava 160 mg (N=48) |
|---|---|---|---|---|
| Mean Age (Years) (SD) | 50.6 (8.3) | 50.9 (8.0) | 52.3 (5.8) | 51.2 (8.3) |
| Sex (Male) (%) | 26 (59%) | 25 (56%) | 30 (61%) | 26 (54%) |
| Race (White) (%) | 40 (91%) | 40 (89%) | 43 (88%) | 46 (96%) |
| Mean Body Mass Index (SD) | 28.8 (3.9) | 26.6 (2.6) | 28.9 (5.3) | 28.8 (5.4) |
| Mean Diastolic BP (SD) | 77.3(8.1) | 78.9(6.1) | 79.0 (8.7) | 76.7 (8.0) |
| Mean Systolic BP (SD) | 118.5 (13.5) | 121.8 (12.9) | 120.6 (14.4) | 120.0 (13.1) |
| Mean TC (mg/dL) (SD) | 267.5 (28.2) | 274.1 (39.6) | 272.8 (34.6) | 274.1 (27.8) |
| Mean LDL-C (mg/dL)^{a} (SD) | 154.4 (29.1) | 165.8 (32.0) | 170.7 (34.2) | 161.0 (30.5) |
| Mean LDL-C (mg/dL)^{b} (SD) | 187.1 (25.7) | 192.8 (30.9) | 195.3 (34.3) | 196.4 (25.0) |
| Mean HDL-C (mg/dL) (SD) | 42.3 (11.0) | 48.8 (13.6) | 41.2 (10.2) | 42.7 (10.6) |
| Mean TG (mg/dL) (SD) | 190.7 (83.5) | 162.3 (77.3) | 181.8 (69.5) | 175.5 (78.9) |

| | | | | |
|---|---|---|---|---|
| *Note:* N = Number of subjects included in the efficacy analyses ^{a} Direct measure ^{b} Calculated | | | | |

There were no clinically significant differences in any of the demographic characteristics or baseline lipid efficacy measures between groups.

### Results:

**Efficacy**: Substantial and clinically significant reductions in LDL-C were seen for all three regimens of pravastatin compared with placebo at 6 weeks of treatment. Pravastatin administered in doses of 40, 80 and 160 mg produced mean reductions in LDL-C of 29%, 37%, and 45%, whereas the mean reduction in the placebo group was minimal (<3%). In addition, a substantial reduction in LDL-C was observed at Weeks 2 and 4 for the pravastatin groups. With one-daily dosing of pravastatin, there was a significant dose-related response (p-value <0.001).

**Results of Efficacy Analysis of LDL-C at Week 6 for Randomized Subjects**

| Lipid | Visit | | Placebo | Prav 40 mg | Prav 80 mg | Prav 160 mg |
|---|---|---|---|---|---|---|
| LDL-C^{a} (mg/dL) | Baseline Week B6 | N | 44 | 45 | 48 | 47 |
| | | Mean | 187.1 | 192.8 | 195.6 | 196.7 |
| | | Mean | 184.1 | 139.0 | 124.7 | 108.4 |
| | | % Δ | -2.6 | -28.6 | -36.7 | -45.2 |
| | | 95% Cl | -7.2, 2.4 | -32.0, -25.0 | -39.6, -33.7 | -47.7, -42.5 |

| | | | | | | |
|---|---|---|---|---|---|---|
| *Note:* N=number or subjects with available efficacy data at Week B6 % Δ is the adjusted percent change from baseline obtained from the ANCOVA model with terms for treatment and baseline ^{a} Calculated | | | | | | |

The treatment effect of pravastatin was also evaluated for HDL-C, TC, and TG at Week 6. Substantial increases in HDL-C of 11.4%, 8.4% and 9.6% were observed for 40, 80, and 160 mg pravastatin, respectively, compared to placebo. At doses of 40, 80 and 160 mg, pravastatin also produced mean reductions in TC of 20%, 26% and 34%, and in TG of 16%, 14% and 27%, respectively. In addition, the treatment effects for HDL-C, TC and TG were evident at Weeks 2 and 4. After 6 weeks of once-daily dosing with pravastatin, there was a positive dose-related response in TC and TG (p-value<0.001). No significant trend was observed for HDL-C.

**Results of Efficacy Analysis of Other Lipids at Week B6 for Randomized Subjects**

| Lipid | Visit | | Placebo | Prav 40 mg | Prav 80 mg | Prav 160 mg |
|---|---|---|---|---|---|---|
| HDL-C (mg/dL) | Baseline Week B6 | N | 44 | 45 | 48 | 48 |
| | | Mean | 42.3 | 48.8 | 40.9 | 42.7 |
| | | Mean | 45.1 | 52.8 | 45.0 | 46.4 |
| | | % Δ | 6.3 | 11.4 | 8.4 | 9.6 |
| | | 95% Cl | 2.1, 10.6 | 7.0, 16.0 | 4.3, 12.7 | 5.4,13.8 |
| TC (mg/dL) | Baseline Week B6 | N | 44 | 45 | 48 | 48 |
| | | Mean | 267.5 | 274.1 | 272.4 | 274.1 |
| | | Mean | 268.4 | 219.8 | 201.6 | 181.3 |
| | | % Δ | -0.1 | -20.0 | -26.3 | -34.2 |
| | | 95% CI | -3.6, 3.5 | -22.8,-17.2 | -28.7, -23.7 | -36.3,-31.9 |
| TG (mg/dL) | Baseline Week B6 | N | 44 | 45 | 48 | 48 |
| | | Mean | 190.7 | 162.3 | 179.5 | 175.5 |
| | | Mean | 196.4 | 140.9 | 158.8 | 132.0 |
| | | %Δ | 1.2 | -15.8 | -14.2 | -26.9 |
| | | 95% Cl | -8.8, 12.3 | -24.0, -6.6 | -22.3, -5.2 | -33.8, -19.2 |

| | | | | | | |
|---|---|---|---|---|---|---|
| *Note:* N=number of subjects with available efficacy data at Week B6 % Δ is the adjusted percent change from baseline obtained from the ANCOVA model with terms for treatment and baseline | | | | | | |

**Safety**: Pravastatin therapy was well tolerated by subjects in this study. The most common treatment-emergent adverse events were headache, upper respiratory infection, musculoskeletal pain, fatigue and abdominal pain (in order of decreasing frequency). The pravastatin 160 mg dose was associated with a higher frequency of adverse events in the gastrointestinal and respiratory body systems. Two subjects (1.4%) in the 160 mg pravastatin treatment group were discontinued due to gastrointestinal events considered to be possibly related to study drug. One placebo-treated subject (2.2%) was discontinued due to headache and fatigue reported as possibly related to study drug. One 40 mg pravastatin-treated subject (2.2%) and one placebo treated subject (2.2%) experienced a CK elevation > 4X ULN, however on-therapy means for CK were lower than baseline means for all treatment groups. No marked abnormalities (MAs) were observed for ALT and AST, nor were there any clinically significant changes in other laboratory parameters. There were no deaths or serious adverse events in the study.

**Number of Randomized Subjects With Adverse Events**

| | Placebo N=46 | Prav 40 mg N=46 | Prav 80 mg N=49 | Prav 160 mg N=48 |
|---|---|---|---|---|
| AE (total) | 20(43.5%) | 19(43%) | 24(49.0%) | 29(60.4%) |
| ADE^{a} | 6(13.0%) | 5(10.9%) | 14(28.6%) | 17(35.4%) |
| SAE | 0 | 0 | 0 | 0 |
| Discontinuations^{a} | 1(2.2%) | 0 | 0 | 2(1.4%) |

| | | | | |
|---|---|---|---|---|
| *Abbreviations:* AE = adverse event, ADE = adverse drug experience, SAE = serious adverse event *Note*: N=Number of randomized subjects included in the analyses. ^{a} Subsets of all AEs; subjects may be represented in more than one group. | | | | |

### Conclusions:

- After 6 weeks of once-daily dosing with pravastatin, there is a clear trend towards greater reduction in LDL-C, TC and TG with increasing doses of 40-160 mg (p-value<0.001), with an additional 8% LDL-C lowering with every doubling of the dose.
- Pravastatin administered once daily for 6 weeks in doses of 80 and 160 mg provide unexpected reductions in LDL-C.
- Pravastatin administered for 6 weeks once daily in doses of 40, 80 and 160 mg to subjects with primary hypercholesterolemia appears safe and well tolerated.

## Claims

1. Use of pravastatin, or a pharmaceutically acceptable salt or ester thereof, and optionally an additional active agent, selected from cholesterol lowering compounds, HMG-COA synthase inhibitors, squalene epoxidase inhibitors, squalene synthetase inhibitors, acyl-coenzyme A, cholesterol acyltransferase (ACAT) inhibitors, probucol, niacin, fibrates, cholesterol absorption inhibitors, bile acid sequestrants, LDL (low density lipoprotein) receptor inducers, vitamin B₆ and the pharmaceutically acceptable salts thereof, vitamin B₁₂, folic acid, platelet aggregation inhibitors, beta-blockers, and anti-oxidant vitamins, for the manufacture of a medicament for reducing LDL cholesterol levels in a mammal, wherein a therapeutically effective amount of 80 mg to 160 mg once per day of pravastatin, or a pharmaceutically acceptable salt or ester thereof is to be administered to a mammal in need of such treatment.

2. The use of Claim 1 wherein the pravastatin is administered once per day in an oral dosage composition.

3. The use of Claim 1 wherein 80 mg of pravastatin is administered once per day.

4. The use of Claim 1 wherein 160 mg of pravastatin is administered once per day

5. The use of Claim 1 wherein pravastatin is administered once per day in a. single oral dosage composition comprising 80 mg of pravastatin and a pharmaceutically acceptable carrier.

6. The use of Claim 1 wherein pravastatin is administered once per day in a single oral dosage composition comprising 160 mg of pravastatin and a pharmaceutically acceptable carrier.

7. A single does composition comprising as active agents 80mg to 160mg of pravastatin or a pharmaceutically acceptable salt thereof, and optionally an additional active agent, selected from cholesterol lowering compounds, HMG-COA synthase inhibitors, squalene epoxidase inhibitors, squalene synthetase inhibitors, acyl-coenzyme A, cholesterol acyltransferase (ACAT) inhibitors, probucol, niacin, fibrates, cholesterol absorption inhibitors, bile acid sequestrants, LDL (low density lipoprotein) receptor inducers, vitamin B₆ and the pharmaceutically acceptable salts thereof, vitamin B₁₂, folic acid, platelet aggregation inhibitors, beta-blockers, and anti-oxidant vitamins.

8. The composition of Claim 9 wherein the single dose is 80 mg.

9. The composition of Claim 9 wherein the single dose is 160 mg.

## Patentansprüche

1. Verwendung von Pravastatin, oder eines pharmazeutisch verträglichen Salzes oder Esters davon, und gegebenenfalls eines zusätzlichen Wirkstoffes, ausgewählt aus Cholesterin senkenden Verbindungen, HMG-COA-Synthasehemmern, Squalenepoxidasehemmern, Squalensynthetasehemmern, Acylcoenzym A, Cholesterinacyltransferase-(ACAT)-Hemmern, Probucol, Niacin, Fibraten, Cholesterinabsorptionshemmern, Gallensäurekomplexbildnern, Induktoren von LDL-(low density lipoprotein)-Rezeptoren, Vitamin B₆ und den pharmazeutisch verträglichen Salzen davon, Vitamin B₁₂, Folsäure, Blutplättchenaggregationshemmern, Betablockern und antioxidierend wirkenden Vitaminen, zur Herstellung eines Medikaments zur Senkung des LDL-Cholesterinspiegels in einem Säuger, wobei eine therapeutisch wirksame Menge an Pravastatin, oder eines pharmazeutisch verträglichen Salzes oder Esters davon, von 80 mg bis 160 mg einmal täglich an einen Säuger, welcher solch einer Behandlung bedarf, verabreicht werden soll.

2. Verwendung gemäß Anspruch 1, wobei das Pravastatin einmal täglich in einer Zusammensetzung für die orale Verabreichung verabreicht wird.

3. Verwendung gemäß Anspruch 1, wobei 80 mg Pravastatin einmal täglich verabreicht werden.

4. Verwendung gemäß Anspruch 1, wobei 160 mg Pravastatin einmal täglich verabreicht werden.

5. Verwendung gemäß Anspruch 1, wobei Pravastatin einmal täglich in einer einzelnen Zusammensetzung für die orale Verabreichung, welche 80 mg Pravastatin und einen pharmazeutisch verträglichen Träger umfasst, verabreicht wird.

6. Verwendung gemäß Anspruch 1, wobei Pravastatin einmal täglich in einer einzelnen Zusammensetzung für die orale Verabreichung, welche 160 mg Pravastatin und einen pharmazeutisch verträglichen Träger umfasst, verabreicht wird.

7. Einzeldosierungszusammensetzung, welche als Wirkstoffe 80 mg bis 160 mg Pravastatin, oder ein pharmazeutisch verträgliches Salz davon, und gegebenenfalls einen weiteren Wirkstoff umfasst, ausgewählt aus Cholesterin senkenden Verbindungen, HMG-COA-Synthasehemmern, Squalenepoxidasehemmern, Squalensynthetasehemmern, Acylcoenzym A, Cholesterinacyltransferase-(ACAT)-Hemmern, Probucol, Niacin, Fibraten, Cholesterinabsorptionshemmern, Gallensäurekomplexbildnern, Induktoren von LDL-(low density lipoprotein)-Rezeptoren, Vitamin B₆ und den pharmazeutisch verträglichen Salzen davon, Vitamin B₁₂, Folsäure, Blutplättchenaggregationshemmern, Betablockern und antioxidierend wirkenden Vitaminen.

8. Zusammensetzung gemäß Anspruch 7, wobei die Einzeldosierung 80 mg beträgt.

9. Zusammensetzung gemäß Anspruch 7, wobei die Einzeldosierung 160 mg beträgt.

## Revendications

1. Utilisation de pravastatine ou d'un de ses sels ou esters pharmaceutiquement acceptables et, facultativement, d'un autre agent actif choisi parmi des composés abaissant le taux de cholestérol, des inhibiteurs de la HMG-CoA synthétase, des inhibiteurs de la squalène époxydase, des inhibiteurs de la squalène synthétase, l'acyl-coenzyme A, des inhibiteurs de la cholestérol acyltransférase (ACAT), le probucol, la niacine, des fibrates, des inhibiteurs de l'absorption du cholestérol, des séquestrants des acides biliaires, des inducteurs des récepteurs de LDL (lipoprotéine de faible densité), la vitamine B₆ et ses sels pharmaceutiquement acceptables, la vitamine B₁₂, l'acide folique, des inhibiteurs de l'agrégation plaquettaire, des bêtabloqueurs et des vitamines antioxydantes, pour la fabrication d'un médicament destiné à réduire les taux de cholestérol LDL chez un mammifère, dans laquelle une quantité thérapeutiquement efficace de 80 mg à 160 mg de pravastatine ou d'un de ses sels ou esters pharmaceutiquement acceptables doit être administrée une fois par jour à un mammifère nécessitant un tel traitement.

2. Utilisation selon la revendication 1, dans laquelle la pravastatine est administrée une fois par jour dans une composition de dose orale.

3. Utilisation selon la revendication 1, dans laquelle 80 mg de pravastatine sont administrés une fois par jour.

4. Utilisation selon la revendication 1, dans laquelle 160 mg de pravastatine sont administrés une fois par jour.

5. Utilisation selon la revendication 1, dans laquelle la pravastatine est administrée une fois par jour dans une composition de dose orale unique comprenant 80 mg de pravastatine et un véhicule pharmaceutiquement acceptable.

6. Utilisation selon la revendication 1, dans laquelle la pravastatine est administrée une fois par jour dans une composition de dose orale unique comprenant 160 mg de pravastatine et un véhicule pharmaceutiquement acceptable.

7. Composition de dose unique comprenant comme agents actifs 80 mg à 160 mg de pravastatine ou d'un de ses sels pharmaceutiquement acceptables et, facultativement, un autre agent actif choisi parmi des composés abaissant le taux de cholestérol, des inhibiteurs de la HMG-CoA synthétase, des inhibiteurs de la squalène époxydase, des inhibiteurs de la squalène synthétase, l'acyl-coenzyme A, des inhibiteurs de la cholestérol acyltransférase (ACAT), le probucol, la niacine, des fibrates, des inhibiteurs de l'absorption du cholestérol, des séquestrants des acides biliaires, des inducteurs des récepteurs de LDL (lipoprotéine de faible densité), la vitamine B₆ et ses sels pharmaceutiquement acceptables, la vitamine B₁₂, l'acide folique, des inhibiteurs de l'agrégation plaquettaire, des bêtabloqueurs et des vitamines antioxydantes.

8. Composition selon la revendication 7, dans laquelle la dose unique est de 80 mg.

9. Composition selon la revendication 7, dans laquelle la dose unique est de 160 mg.
